# FASCICULE DE BREVET EUROPEEN

(11) **EP 1 381 364 B1**
(45) Date de publication et mention de la délivrance du brevet: **23.08.2006**
(21) Numéro de dépôt: 02735506.4
(22) Date de dépôt: 26.04.2002
(51) Int. Cl.: A61K 31/443, A61K 33/32, A61P 1/16

(54) **UTILISATION DU MANGAFODIPIR DANS LE TRAITEMENT DE L'INSUFFISANCE HÉPATOCELLULAIRE**
VERWENDUNG VON MANGAFODIPIR ZUR BEHANDLUNG DER HEPATOZELLULAREN INSUFFIZIENZ
USE OF MANGAFODIPIR FOR TREATING HEPATOCELLULAR INSUFFICIENCY

(30) Priorité: 26.04.2001 FR 0105606
(43) Date de publication de la demande: 21.01.2004
(73) Titulaire: UNIVERSITE RENE DESCARTES, (PARIS V), F-75006 Paris (FR)
(72) Inventeur: BATTEUX, Frédéric, F-75015 Paris (FR); WEILL, Bernard, F-95600 Eaubonne (FR)
(74) Mandataire: Vialle-Presles, Marie José
(86) Numéro de dépôt international: PCT/FR2002/001457
(87) Numéro de publication internationale: WO 2002/087579

(56) Documents cités:
- WO-A-97/49409
- WO-A-99/33521
- BRUROK, HEIDI ET AL: "Manganese dipyridoxyl diphosphate: MRI contrast agent with antioxidative and cardioprotective properties? in vitro and ex vivo assessments" BIOCHEM. BIOPHYS. RES. COMMUN. (1999), 254(3), 768-772, XP001058319
- MARCHAL, G. ET AL: "Comparison between gadolinium-DTPA, gadolinium-EOB-DPTA, and manganese-DPDP in induced HCC in rats: a correlation study of MR imaging, microangiography, and histology" MAGN. RESON. IMAGING (1993), 11(5), 665-74, XP001052675
- OUDKERK M ET AL: "Better liver lesion diagnosis with mangafodipir trisodium (MnDPDP)-enhanced MR liver imaging: A multicenter study comparing MR and biphasic spiral CT." JOURNAL OF HEPATOLOGY, vol. 32, no. Supplement 2, 2000, page 136 XP001058294 35th Annual Meeting of the European Association for the Study of the Liver;Rotterdam, Netherlands; April 29-May 03, 2000 ISSN: 0168-8278
- ASPLUND A ET AL: "MANGAFODIPIR (MNDPDP)- AND MNCL2-INDUCED ENDOTHELIUM-DEPENDENT RELAXATION IN BOVINE MESENTRIC ARTERIES. 1" JOURNAL OF PHARMACOLOGY AND EXPERIMENTAL THERAPEUTICS, AMERICAN SOCIETY FOR PHARMACOLOGY AND, US, vol. 271, no. 2, 1 novembre 1994 (1994-11-01), pages 609-614, XP002043389 ISSN: 0022-3565
- YOUNG, STUART W. ET AL: "Detection of hepatic malignancies using manganese-DPDP (manganese dipyridoxal diphosphate) hepatobiliary MRI contrast agent" MAGN. RESON. IMAGING (1990), 8(3), 267-76, XP001052679
- GRAUL, A. ET AL: "Mangafodipir trisodium" DRUGS FUTURE (1997), 22(9), 974-979, XP001058112

## Description

L'invention est relative à l'utilisation du Mangafodipir dans le cadre du traitement des insuffisances hépatocellulaires.

Le manganèse dipyridoxyl phosphate (Mn-DPDP), également dénommé Mangafodipir (DCI), est utilisé en radiologie comme produit de contraste paramagnétique dans le cadre du diagnostic par IRM (imagerie par résonnance magnétique) ; pour revue sur les propriétés du Mangafodipir, cf. ROCKLAGE *et al.*, Inorg. Chem., 28, 477-485, (1989).

Certaines propriétés pharmacologiques du Mangafodipir ont également été rapportées : ASPLUND et al., (J. Pharmacol. Exp. Therapeutics, vol.271, no.2, p.609-614, 1994) décrivent ses propriétés vaso-dilatatrices, attribuées à un effet de stabilisation du monoxyde d'azote produit par les cellules de l'endothélium vasculaire ; ces auteurs indiquent que cet effet pourrait découler d'une activité de type superoxyde dismutase (SOD). BRUROK et al. (Biochem. Biophys. Res. Commun., vol.254, no.3, p.768-772, 1999) ont observé *in vitro* ses propriétés de mimétique de la SOD, se traduisant *ex vivo* par une action cardioprotectrice vis-à-vis des effets des radicaux oxygénés libres O₂⁻ et OH⁻ générés lors d'une hypoxie suivie de réoxygénation. La Demande PCT WO 97/49409 propose l'utilisation du Mangafodipir ou d'autres agents chélatants dérivés de dipyridoxyle ou d'acide aminopolycarboxylique dans le cadre du traitement de pathologies induites par les radicaux libres, à savoir les lésions résultant d'une ischémie-reperfusion, intervenant notamment au cours de l'infarctus ou d'autres maladies cardiovasculaires, ou les pathologies pro-inflammatoires telles que les lésions induites par les radiations. La Demande PCT WO 99/33521 propose l'utilisation de ces mêmes agents chélatants pour traiter l'athérosclérose, en prévenant l'oxydation des LDL (low density lipoproteins) ou comme agents cytotoxiques pour traiter des infections par des bactéries ou des protozoaires, ou comme agents détoxifiants pour le traitement d'intoxications par des métaux tels que le fer.

Les diverses utilisations thérapeutiques du Mangafodipir proposées ci-dessus découlent principalement de ses propriétés de mimétique de superoxyde dismutase. La superoxyde dismutase (EC 1.15.1.1) intervient dans la détoxification des radicaux oxygénés libres, en catalysant la dismutation de l'anion superoxyde (O₂⁻) en peroxyde d'hydrogène (H₂O₂). On distingue différents types de superoxyde dismutase, parmi lesquels on citera notamment les superoxyde dismutases à cuivre et zinc (CuZnSOD), également connues sous la désignation superoxyde dismutase-1, qui, chez les organismes eucaryotes, sont principalement localisées dans le cytoplasme, et les superoxyde dismutases à manganèse (MnSOD), également connues sous la désignation superoxyde dismutase-2, que l'on trouve principalement chez les procaryotes et dans les organites intracellulaires des cellules eucaryotes.

L'utilisation de mimétiques de la SOD a été proposée dans le cadre de diverses pathologies impliquant un stress oxydant provoqué par les radicaux oxygénés libres [pour revue cf. PATEL et DAY, Trends Pharmacol. Sci., 20, 359-364 (1999)]. Cependant, leur efficacité est inégale selon la pathologie concernée.

Ainsi, lors de travaux antérieurs concernant l'insuffisance hépatocellulaire, qui est une des nombreuses pathologies dans laquelle un rôle potentiel des radicaux oxygénés libres a été évoqué, les Inventeurs ont observé qu'un mimétique de la CuZnSOD, le CuDIPS (Cu[II]-[diisopropylsalicylate]), n'avait aucune effet significatif ; en revanche, ils ont constaté qu'un mimétique de la MnSOD, le MnTBAP [Mn(III)-tetrakis-(5,10,15,20-benzoic acid)-porphyrin], possédant également une activité catalase et une activité glutathion peroxydase, avait un effet préventif et curatif notable (Demande PCT/WO 01/12327 ; FERRET et al., Hepatology, vol.33, no.5, p.1173-1180, mai 2001).

Cette efficacité du MnTBAP pourrait s'expliquer par ses activités catalase et glutathion peroxydase, qui complèteraient son activité SOD en permettant la détoxification du peroxyde d'hydrogène généré par dismutation de l'anion superoxyde.

Pour vérifier cette hypothèse, les Inventeurs ont entrepris de rechercher d'autres mimétiques de la SOD possédant des activités de détoxification d'espèces oxygénées réactives autres que l'anion superoxyde, et de tester leur effet sur l'insuffisance hépatocellulaire.

Ils ont ainsi constaté que, contrairement à ce qui avait été précédemment rapporté (BRUROK et al., 1999 précité), le Mangafodipir possédait comme le MnTBAP, une activité catalase, et qu'il possédait en outre une activité glutathion réductase. Ils ont aussi observé que son efficacité vis-à-vis de l'insuffisance hépatocellulaire était au moins égale à celle du MnTBAP.

La présente invention a pour objet l'utilisation du Mangafodipir pour l'obtention d'un médicament destiné au traitement préventif ou curatif d'une insuffisance hépatocellulaire.

On désigne sous le terme d'insuffisance hépatocellulaire un ensemble de manifestations pathologiques résultant de la destruction des hépatocytes. Selon l'étendue de la destruction cellulaire, ces manifestations cliniques sont plus ou moins graves et réversibles. Dans des cas extrêmes, la destruction massive et subite des hépatocytes aboutit à une insuffisance hépatique aiguë, également dénommée hépatite fulminante, qui peut entraîner la mort en quelques jours.

Parmi les causes les plus fréquentes de destruction des hépatocytes pouvant entraîner une insuffisance hépatocellulaire on mentionnera notamment les infections virales, dues aux différents types de virus de l'hépatite, ainsi que les intoxications, notamment par certains médicaments ou par l'alcool.

On dispose actuellement de plusieurs modèles animaux d'insuffisance hépatocellulaire de différentes origines, qui permettent notamment d'induire expérimentalement une insuffisance hépatique aiguë, et d'étudier les mécanismes aboutissant à la destruction cellulaire. Différents mécanismes impliquant des radicaux oxygénés libres ont ainsi été proposés.

Par exemple, l'insuffisance hépatique aiguë d'origine toxique, induite notamment par l'acétaminophène, résulte d'une saturation des mécanismes normaux de détoxication hépatique. En effet, aux doses pharmacologiques, l'acétaminophène est principalement éliminé par glucoro- et sulfo-conjugaison, mais est également oxydé par le cytochrome P450 en N-acétyl-p-benziquinone-imine (NAPQI), qui peut normalement être ensuite éliminée après conjugaison avec le glutathion. En cas de surdosage, on observe la saturation des voies de glucoro- et sulfo-conjugaison, et une production accrue de NAPQI [PRESCOTT, Drugs, 25, 290-314, (1983)]. Ce métabolite très réactif est supposé être l'effecteur principal des lésions des hépatocytes, et les traitements médicamenteux proposés sont basés essentiellement sur l'utilisation d'antioxydants, tels que la N-acétyl-L-cystéine, qui ont pour but de permettre la reconstitution des réserves intracellulaires de glutathion et la neutralisation du NAPQI. L'efficacité de ces traitements est toutefois inconstante [CARACENI et VAN THIEL, Lancet, 345, 163-169, (1995) ; SCHIODT *et al.,* N. Engl. J. Med., 337, 1112-1117, (1997)], et en cas d'hépatite fulminante, la greffe du foie constitue actuellement le seul traitement réellement efficace.

Dans le cas de l'insuffisance hépatocellulaire induite par l'alcool, plusieurs mécanismes ont été proposés, faisant intervenir notamment la génération de métabolites toxiques comme l'acétaldéhyde, l'anion superoxyde ou le peroxyde d'hydrogène.

Les Inventeurs ont testé l'effet du Mangafodipir sur l'insuffisance hépatique aiguë d'origine toxique, en utilisant un modèle expérimental d'insuffisance hépatique aiguë induite par administration d'acétaminophène. Ils ont ainsi constaté que l'administration du Mangafodipir permet d'augmenter très significativement le taux de survie après administration d'une dose létale d'acétaminophène, et d'en réduire considérablement les effets toxiques. Il apparaît donc que le Mangafodipir protége très efficacement les hépatocytes des effets destructeurs de substances toxiques, et exerce ainsi un effet à la fois préventif et curatif sur l'insuffisance hépatocellulaire, notamment d'origine toxique (médicamenteuse ou induite par l'alcool).

En outre, les Inventeurs ont constaté que les effets bénéfiques du Mangafodipir sont observés non seulement lorsque celui-ci est administré à titre préventif, mais également lorsqu'il est administré à titre curatif, c'est à dire après l'apparition des premiers effets hépatotoxiques.

Selon un mode de mise en oeuvre préféré de la présente invention, le Mangafodipir est utilisé pour l'obtention d'un médicament destiné au traitement préventif ou curatif d'une insuffisance hépatocellulaire d'origine toxique, et notamment au traitement d'une insuffisance hépatocellulaire induite par l'acétaminophène, ou d'une insuffisance hépatocellulaire induite par l'alcool.

Le Mangafodipir peut en particulier être utilisé pour l'obtention d'un médicament destiné au traitement d'une insuffisance hépatocellulaire aiguë, se manifestant notamment sous forme d'une hépatite fulminante.

L'activité glutathion réductase du Mangafodipir qui permet de régénérer le pool intracellulaire de glutathion, complète avantageusement son activité SOD et son activité catalase, notamment dans le cadre du traitement de l'insuffisance hépatique aiguë d'origine toxique, par exemple celle induite par l'acétaminophène.

Pour la mise en oeuvre de la présente invention, le Mangafodipir sera généralement employé dans des formulations permettant l'administration d'une dose de principe actif comprise entre 0,1 et 10 mg/kg/jour (administration de façon préventive) ou entre 5 et 50 mg/kg/jour (administration de façon curative) ; des doses plus élevées peuvent toutefois être utilisées, compte tenu de la faible toxicité de ce produit. Il est bien entendu que l'homme de l'art peut adapter ces doses en fonction des particularités de chaque patient et de la pathologie concernée.

Dans le cadre de la mise en oeuvre de la présente invention, le Mangafodipir peut être administré par différentes voies. Généralement, il sera administré par voie orale, ou par injections, en particulier par injections sous-cutanées, intra-musculaires ou intra-veineuse. D'autres voies d'administration pourront être envisagées si elles augmentent l'efficacité, la biodisponibilité ou la tolérance des produits.

La présente invention sera mieux comprise à l'aide du complément de description qui va suivre, qui se réfère à des exemples non-limitatifs démontrant les activités SOD, catalase, et gluthation réductase du Mangafodipir et ses effets *in vitro* et *in vivo* sur l'insuffisance hépatocellulaire.

### EXEMPLE 1 : ACTIVITE MIMETIQUE DE LA SOD DU MANGAFODIPIR.

L'activité SOD d'un composé chimique peut être déterminée par la méthode de réduction du NBT (nitro bleu de tétrazolium) selon la technique décrite par BEAUCHAMP et FRIDOVICH [Anal. Biochem., 44(1), 276-87, (1971)].

Le test est réalisé à 25°C dans un volume final de tampon de 0,8 ml (50mM TRIS/HCl, pH 7,6), contenant 22 µM de xanthine, 500 U/ml de catalase et 0,2 U/ml de xanthine oxydase. La réduction du NBT, en présence de 1,5 µg de Mangafodipir, ou à titre de contrôle, en l'absence de Mangafodipir, est mesurée toutes les 30 secondes pendant 5 minutes. Une unité SOD (U SOD) est définie par la quantité d'enzyme capable d'inhiber le taux de réduction du NBT de 50%.

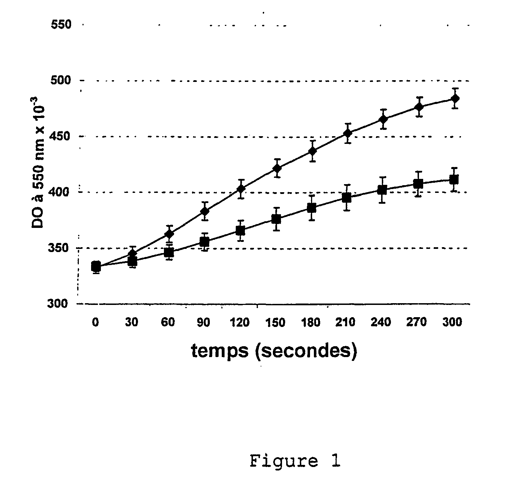

Les résultats sont illustrés dans la figure 1. Légende de la Figure 1 :
◆ : Contrôle
■ : Mangafodipir (1,5 µg)

Ils montrent que la vitesse de réduction du NBT passe de 0,0302/minute à 0,01556 lorsque 1,5 µg de Mangafodipir est ajouté dans le mélange réactionnel. A partir de ces résultats, l'activité SOD du Mangafodipir a été évaluée à 680 U SOD par milligramme de Mangafodipir.

### EXEMPLE 2 : ACTIVITE MIMETIQUE DE LA CATALASE DU MANGAFODIPIR.

Le dosage de l'activité catalase a été réalisé selon la méthode décrite par AEBI [Methods Enzymol., 105, 121-126, (1984)]. La décroissance de la densité optique (DO) à 240 nm d'une solution d'H₂O₂ 10 mM en tampon phosphate (50 mM Na₂HPO₄/KH₂PO₄, 0,1 mM EDTA, pH 7,4) a été mesurée en présence de 75,7 µg de Mangafodipir, ou à titre de référence, en présence de 1 unité de catalase bovine.

Dans ces conditions, l'activité catalase du Mangafodipir a été évaluée à 2 U catalase par milligramme de Mangafodipir.

### EXEMPLE 3 : ACTIVITE MIMETIQUE DE LA GLUTHATION REDUCTASE DU MANGAFODIPIR.

Le dosage de l'activité gluthation réductase à été réalisé à l'aide de la trousse: « Gluthathione reductase assay kit » commercialisée par la société CALBIOCHEM. Le mélange réactionnel est analysé par spectrophotométrie à 340nm pendant 5 minutes.

L'activité gluthation réductase du Mangafodipir a été évaluée à 19,9 ± 1,75 mU gluthation réductase par milligramme de Mangafodipir.

### EXEMPLE 4 : ACTIVITE PROTECTRICE DU MANGAFODIPIR SUR DES HEPATOCYTES EN CULTURE SOUMIS A L'ACTION DE L'ANION SUPEROXYDE O₂⁻.

La lignée de cellules hépatiques humaines Hep 3B a été utilisée pour ces expériences.

Les cellules ont été ensemencées dans une plaque de culture de 96 puits, à raison de 5x10⁴ cellules par puits, dans un volume final de 50 µl.

Cent µl de solution de Mangafodipir à différentes concentrations (62,5, 125, 250 µg/ml) ont été déposés dans chaque puits (trois puits par concentration testée). Après une heure d'incubation, 50 µl d'une solution de xanthine à 200 µM et de 2 U/ml de xanthine oxydase (solution X/XO) ont été déposés dans chaque puits. Dix puits n'ont pas reçu de solution X/XO et ont servi de témoins pour évaluer la viabilité de base des cellules. Douze heures après le dépôt de la solution X/XO, les cellules ont été lavées avec une solution saline et la viabilité déterminée grâce à un test au MTT. Une solution de MTT 0,2% a été déposée dans chaque puits et incubée 4 heures à 37°C. Ensuite, les cellules ont été lavées et le test révélé par addition dans chaque puits de 50 µl de Diméthylsulfoxyde (DMSO). Les plaques ont ensuite été lues au spectrophotomètre à une longueur d'onde de 550 nm.

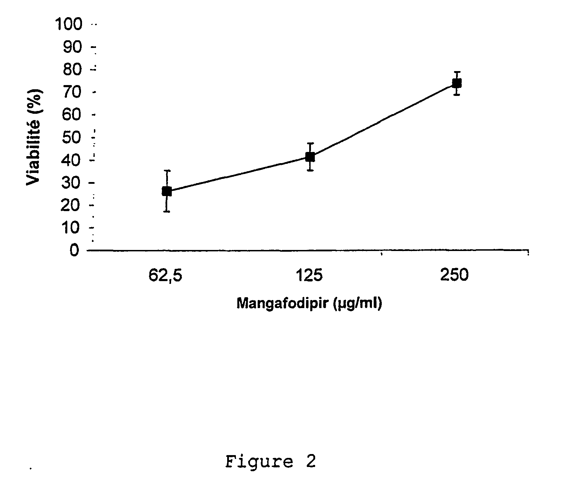

Les résultats (pourcentage de viabilité en fonction de la concentration en Mangafodipir) sont illustrés par la figure 2.

Ces résultats montrent que l'addition de Mangafodipir prévient la mortalité des cellules hépatiques soumises à un stress oxydant médié par l'anion superoxyde.

### EXEMPLE 5 : ACTIVITE PROTECTRICE DU MANGAFODIPIR SUR DES HEPATOCYTES EN CULTURE SOUMIS A L'ACTION DU PEROXYDE D'HYDROGENE (H₂O₂).

La lignée de cellules hépatiques humaines Hep 3B a également été utilisée pour ces expériences.

Les cellules ont été ensemencées dans une plaque de culture de 96 puits, à raison de 5x10⁴ cellules par puits, dans un volume final de 50 µl.

Cent µl de solution de Mangafodipir à différentes concentrations (62,5, 125, 250 µg/ml) ont été déposés dans chaque puits (trois puits par concentration testée). Après une heure d'incubation, 50µl d'une solution d'H₂O₂ à 4mM ont été déposés dans chaque puits. Dix puits n'ont pas reçu d'H₂O₂ et servent de témoins pour évaluer la viabilité de base des cellules. Douze heures après le dépôt de la solution d'H₂O₂, les cellules ont été lavées avec une solution saline et la viabilité déterminée grâce à un test au MTT, comme décrit dans l'exemple 4 ci-dessus.

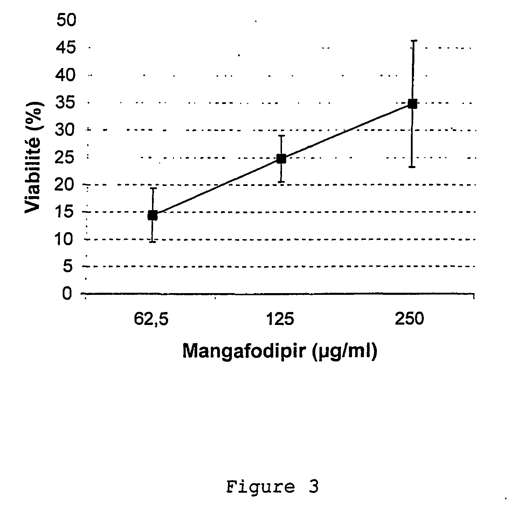

Les résultats (pourcentage de viabilité en fonction de la concentration en Mangafodipir) sont illustrés par la figure 3.

Ces résultats montrent que l'addition de Mangafodipir prévient la mortalité des cellules hépatiques soumis à un stress oxydant médié par le peroxyde d'hydrogène.

### EXEMPLE 6 : ACTIVITE DU MANGAFODIPIR SUR L'INSUFFISANCE HEPATIQUE AIGUË INDUITE PAR L'ACETAMINOPHENE.

L'injection intrapéritonéale d'acétaminophène chez la souris induit une hépatotoxicité sévère, dont le degré peut être évalué par la survie des animaux, et l'examen macroscopique et microscopique des foies.

### Survie des animaux

Le Mangafodipir, (commercialisé sous le nom de TESLASCAN par Nicomed-Amersham) est administré sous forme de bolus, par voie intrapéritonéale.

L'acétaminophène (APAP), en solution à 100 mg/ml dans du PBS à pH 7,4 est administré par voie intrapéritonéale.

Dans une première série d'expérimentations, un groupe de souris a reçu une dose de 1000 mg/kg d'acétaminophène ; un second groupe a reçu une dose de 1000 mg/kg d'acétaminophène, et une dose de 10 mg/kg de Mangafodipir administrée soit 2 h avant l'acétaminophène, soit 6 h après ; un groupe témoin a reçu soit du Mangafodipir seul (10 mg/kg), soit du PBS seul.

La survie des animaux est suivie pendant 24 heures après l'administration d'acétaminophène.
Groupe I : PBS
Groupe II : APAP 1000 mg/kg
Groupe III : APAP 1000 mg/kg + Mangafodipir 10 mg/kg préventif
Groupe IV : APAP 1000 mg/kg + Mangafodipir 10 mg/kg Curatif

Les résultats (nombre d'animaux survivants) sont résumés dans le tableau I ci-après :

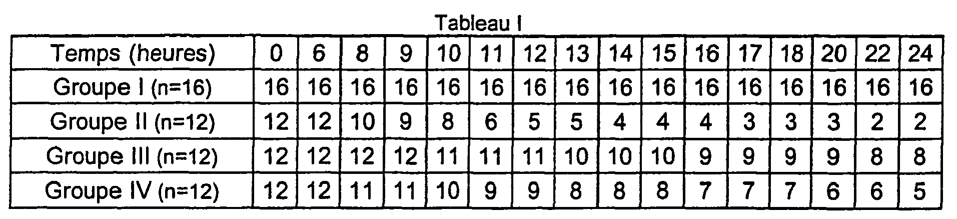

Ces résultats montrent que 24 heures après l'injection d'acétaminophène, 16% des animaux intoxiqués par l'APAP à la dose de 1000 mg/kg sont morts, alors que le taux de survie est supérieur à 41% chez les animaux intoxiqués par l'APAP mais ayant reçu le Mangafodipir après l'administration d'acétaminophène (protocole curatif), et de l'ordre de 66% chez les animaux ayant reçu le Mangafodipir avant l'administration d'acétaminophène (protocole préventif). Aucun décès n'est observé chez les animaux ayant reçu le PBS seul.

### Etude histologique

Dans chacun des groupes, les foies de plusieurs animaux ont été prélevés, pour effectuer une étude histologique. Dans le cas des souris ayant reçu de l'acétaminophène on observe beaucoup moins de lésions apoptotiques chez les animaux traités au Mangafodipir que chez les animaux non traités. Aucune lésion apoptotique n'est visible chez les souris du groupe témoin n'ayant pas reçu d'acétaminophène.

### EXEMPLE 7 : COMPARAISON DES ACTIVITES DU MANGAFODIPIR, DU MNTBAP, ET DU CUDIPS SUR L'INSUFFISANCE HEPATIQUE AIGUË INDUITE PAR L'ACETAMINOPHENE.

Le CuDIPS (Cu[II]-[diisopropylsalicylate]) est un mimétique de référence de la CuZnSOD (Mc KENZIE *et al*., Br. J. Pharmacol. 127, 1159-1164, (1999)] ; le MnTBAP (Mn(III) tetrakis (5,10,15,20-benzoic acid) porphyrin) est un mimétique de la MnSOD, possédant également une activité catalase et une activité glutathion peroxydase (Demande PCT/WO 01/12327). L'effet du CuDIPS et du MnTBAP sur la survie de souris après injection intrapéritonéale de 1000 mg/kg d'acétaminophène (APAP) a été comparé à celui du Mangafodipir.

Le protocole expérimental est le suivant :

Le Mangafodipir, le MnTBAP ou le CuDIPS sont administrés sous forme de bolus, à titre préventif, 2 heures avant l'acétaminophène.

Différents groupes d'animaux ont reçu les traitements suivants :
Groupe I : PBS
Groupe II : APAP 1000 mg/kg
Groupe III : APAP 1000 mg/kg ; MnTBAP 10 mg/kg
Groupe IV : APAP 1000 mg/kg ; CuDIPS 10 mg/kg
Groupe V : APAP 1000 mg/kg ; Mangafodipir 10 mg/kg

La survie des animaux est suivie pendant 24 heures après l'administration d'acétaminophène.

Les résultats, exprimés en nombre d'animaux survivants sont illustrés par le tableau II ci après.

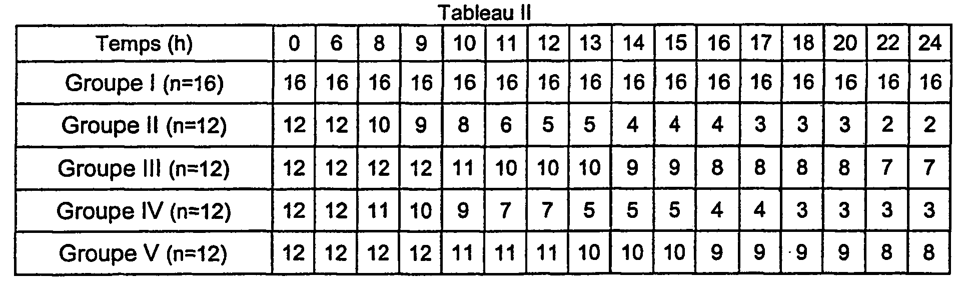

Ces résultats montrent que :
- le Mangafodipir administré préventivement augmente le taux de survie de manière au moins égale au MnTBAP ;
- le CuDIPS n'augmente pas le taux de survie de manière significative.

### Dosage des transaminases

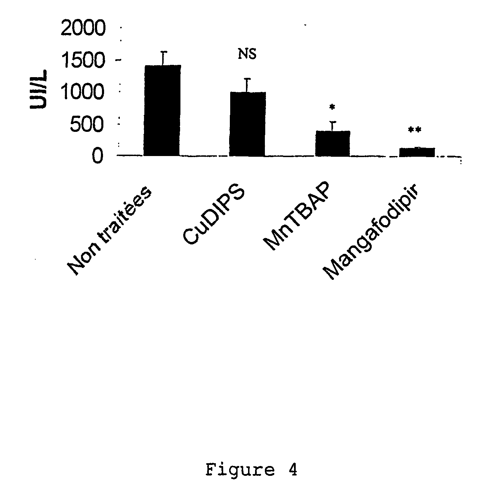

Dans une seconde série d'expériences, un groupe de souris a reçu une dose de 500 mg/kg d' acétaminophène : un second groupe a reçu la même dose d'acétaminophène, et une dose de 10 mg/kg de Mangafodipir administrée 2 h avant l'acétaminophène ; un troisième groupe d'animaux a reçu la même dose d'acétaminophène, et une dose de 10 mg/kg de MnTBAP, un quatrième groupe d'animaux a reçu la même dose d'acétaminophène, et une dose de 10 mg/kg de CuDIPS. Les transaminases sériques ASAT sont dosées 12 heures après l'administration d'acétaminophène.
Les résultats sont illustrés par la figure 4.
Légende de la figure 4 :
NS : non significatif par rapport aux souris non traitées
* : P<0.01 par rapport aux souris non traitées
** : P<0.001 par rapport aux souris non traitées

Parmi les souris ayant reçu 500 mg/kg d'acétaminophène (APAP₅₀₀), on observe, après 12 heures, des activités transaminases 10 fois supérieures à chez celles qui ont reçu préalablement un traitement par le Mangafodipir.

Ces résultats montrent que dans tous les cas, l'administration de Mangafodipir réduit les activités transaminases, qui reflètent la cytolyse hépatique.

## Revendications

1. Utilisation du Mangafodipir pour l'obtention d'un médicament destiné au traitement préventif ou curatif d'une insuffisance hépatocellulaire.

2. Utilisation selon la revendication 1, **caractérisée en ce que** ladite insuffisance hépatocellulaire est d'origine toxique.

3. Utilisation selon la revendication 2, **caractérisée en ce que** ladite insuffisance hépatocellulaire est induite par l'acétaminophène.

4. Utilisation selon la revendication 2, **caractérisée en ce que** ladite insuffisance hépatocellulaire est induite par l'alcool.

5. Utilisation selon une quelconque des revendications 1 à 4, **caractérisée en ce que** ladite insuffisance hépatocellulaire se manifeste sous forme d'une hépatite fulminante.

6. Utilisation selon une quelconque des revendications 1 à 5, **caractérisée en ce que** le Mangafodipir est utilisé pour l'obtention d'un médicament destiné à un traitement préventif.

7. Utilisation selon une quelconque des revendications 1 à 5, **caractérisée en ce que** le Mangafodipir est utilisé pour l'obtention d'un médicament destiné à un traitement curatif.

8. Utilisation selon la revendication 6, **caractérisée en ce que** ledit médicament est formulé pour permettre l'administration d'une dose de Mangafodipir comprise entre 0,1 et 10 mg/kg/jour.

9. Utilisation selon la revendication 7, **caractérisée en ce que** ledit médicament est formulé pour permettre l'administration d'une dose de Mangafodipir comprise entre 5 et 50 mg/kg/jour.

## Claims

1. The use of Mangafodipir for producing a medicinal product intended for the preventive or curative treatment of hepatocellular deficiency.

2. The use as claimed in claim 1, **characterized in that** said hepatocellular deficiency is of toxic origin.

3. The use as claimed in claim 2, **characterized in that** said hepatocellular deficiency is induced by acetaminophen.

4. The use as claimed in claim 2, **characterized in that** said hepatocellular deficiency is induced by alcohol.

5. The use as claimed in any one of claims 1 to 4, **characterized in that** said hepatocellular deficiency manifests itself in the form of fulminant hepatitis.

6. The use as claimed in any one of claims 1 to 5, **characterized in that** the Mangafodipir is used for producing a medicinal product intended for a preventive treatment.

7. The use as claimed in any one of claims 1 to 5, **characterized in that** the Mangafodipir is used for producing a medicinal product intended for a curative treatment.

8. The use as claimed in claim 6, **characterized in that** said medicinal product is formulated for administering a dose of Mangafodipir in a range comprised between 0.1 and 10 mg/kg/day.

9. The use as claimed in claim 7, **characterized in that** said medicinal product is formulated for administering a dose of Mangafodipir in a range comprised between 5 and 50 mg/kg/day.

## Patentansprüche

1. Verwendung von Mangafodipir, um ein Medikament zu erhalten, das zur vorbeugenden oder heilenden Behandlung einer hepatozellulären Insuffizienz bestimmt ist.

2. Verwendung nach Anspruch 1, **dadurch gekennzeichnet, dass** die hepatozelluläre Insuffizienz toxischen Ursprungs ist.

3. Verwendung nach Anspruch 2, **dadurch gekennzeichnet, dass** die hepatozelluläre Insuffizienz durch Acetaminophen induziert wurde.

4. Verwendung nach Anspruch 2, **dadurch gekennzeichnet, dass** die hepatozelluläre Insuffizienz durch Alkohol induziert wurde.

5. Verwendung nach einem der Ansprüche 1 bis 4, **dadurch gekennzeichnet, dass** die hepatozelluläre Insuffizienz sich in Form einer fulminanten Hepatitis manifestiert.

6. Verwendung nach einem der Ansprüche 1 bis 5, **dadurch gekennzeichnet, dass** Mangafodipir verwendet wird, um ein Medikament zu erhalten, das zur vorbeugenden Behandlung bestimmt ist.

7. Verwendung nach einem der Ansprüche 1 bis 5, **dadurch gekennzeichnet, dass** Mangafodipir verwendet wird, um ein Medikament zu erhalten, das zur heilenden Behandlung bestimmt ist.

8. Verwendung nach Anspruch 6, **dadurch gekennzeichnet, dass** das Medikament so formuliert ist, dass die Verabreichung einer Dosis von Mangafodipir zwischen 0,1 und 10 mg/kg/Tag möglich ist.

9. Verwendung nach Anspruch 7, **dadurch gekennzeichnet, dass** das Medikament so formuliert ist, dass die Verabreichung einer Dosis von Mangafodipir zwischen 5 und 50 mg/kg/Tag möglich ist.
